# FASCICULE DE BREVET EUROPEEN

(11) **EP 1 383 816 B1**
(45) Date de publication et mention de la délivrance du brevet: **11.07.2007**
(21) Numéro de dépôt: 02730345.2
(22) Date de dépôt: 04.04.2002
(51) Int. Cl.: C08G 18/08, C08G 18/66, C09D 175/04, C08K 3/34

(54) **COMPOSITION FILMOGENE A USAGE TOPIQUE ET SON UTILISATION POUR LA DELIVRANCE D'AGENTS ACTIFS**
FOLIENZUSAMMENSETZUNG ZUR LOKALEN ANWENDUNG UND VERWENDUNG ZUR FREISETZUNG VON AKTIVSTOFFEN
FILM-FORMING COMPOSITION FOR TOPICAL USE AND USE THEREOF FOR DELIVERING ACTIVE AGENTS

(30) Priorité: 04.04.2001 FR 0104582
(43) Date de publication de la demande: 28.01.2004
(73) Titulaire: Lavipharm Laboratories, Inc., East Windsor, NJ 08520 (US)
(72) Inventeur: ZOLOTARSKY, Yelena, Springfield, NJ 07081 (US); O'HALLORAN, David, US-Milltown, NJ 08850 (US); GUILLIER-BERNARD, Florence, F-75017 Paris (FR)
(74) Mandataire: Breese, Pierre
(86) Numéro de dépôt international: PCT/FR2002/001182
(87) Numéro de publication internationale: WO 2002/081536

(56) Documents cités:
- WO-A-01/10397
- GB-A- 2 105 732
- US-A- 5 120 529
- US-A- 6 106 813

## Description

L'invention concerne une nouvelle composition cosmétique et/ou thérapeutique, filmogène à usage topique. L'invention concerne également l'utilisation de cette composition filmogène pour la délivrance d'agents actifs au niveau de la peau.

De nombreuses compositions cosmétiques ou thérapeutiques permettent d'appliquer au niveau de la peau des agents actifs cosmétiques ou thérapeutiques. Cependant, une application au niveau de la peau doit résister aux conditions environnementales et aux frottements avec les vêtements. Les compositions standard actuellement utilisées résistent mal et leur application doit être répétée pour obtenir l'effet désiré (maquillage ou traitement thérapeutique). Par exemple, en cosmétologie, l'utilisation de cellulose et d'argile pour mettre en suspension des pigments ou des particules est une pratique courante mais le film produit est très facilement éliminé en frottant avec de l'eau. De plus, ce type de films est également très sec et très poudreux.

Les travaux de la Demanderesse ont permis de mettre au point une composition filmogène à usage topique pour résoudre les problèmes ci-dessus. Cette composition, lors de son application sur la peau, sèche pour donner un film distinctif, visible ou invisible. Ce film présente également un aspect lisse uniforme. Il est confortable à porter, résiste mieux aux conditions environnementales comme la pluie et aux frottements légers, comparé aux produits déjà connus dans l'art antérieur.

Cette nouvelle composition filmogène à usage topique est remarquable car elle constitue un nouveau mode de délivrance d'agents actifs cosmétiques ou thérapeutiques au niveau de la peau. En effet, grâce aux propriétés de résistance du film produit, les agents actifs cosmétiques ou thérapeutiques qu'elle contient restent plus longtemps actifs au niveau de la peau.
La composition filmogène à usage topique objet de l'invention est un système polymérique et est caractérisée en ce qu'elle contient au moins du polyuréthane, de la cellulose et du silicate de magnésium et d'aluminium.

Le polyuréthane avantageusement utilisé dans la composition objet de la présente invention est du polyuréthane-1.
Le polyuréthane-1 est un copolymère de monomères d'acide isophthalique, d'acide adipique, de glycol héxylène, de glycol néopentyl, d'acide diméthylolpropanique et de diisocyanate d'isophorone. Ce polymère est déjà connu pour sa capacité à former une pellicule. Il est notamment utilisé dans les produits fixants pour le soin des cheveux et dans le mascara. Les travaux de la Demanderesse ont notamment permis de mettre en évidence sa capacité à libérer au niveau de la peau des agents actifs. La cellulose est déjà utilisée pour sa capacité à former des pellicules et à libérer des agents actifs au niveau de la peau mais n'a jamais été utilisée en combinaison avec le polyuréthane.
Le silicate de magnésium et d'aluminium est également utilisé comme stabilisateur et comme agent modifiant la viscosité mais n'a jamais été associé dans une combinaison synergique avec le polyuréthane et la cellulose.

Pris individuellement, chacun de ces composés a tendance à sécher et à s'écailler. La composition mise au point par la Demanderesse est unique car elle présente une durée accrue, le film produit restant stable plus longtemps.
De plus, la pulvérisation de cellulose à partir d'un aérosol ou d'un pulvérisateur à pompe est généralement impossible. Avec la composition polymérique de l'invention, une brume uniforme est pulvérisée.

Les travaux de la Demanderesse ont également permis de mettre en évidence les proportions les meilleures des trois composés à utiliser dans la composition filmogène selon l'invention. Ainsi, la composition filmogène selon l'invention contient avantageusement :
- de 1 à 50 %, et de préférence de 3 à 30 % en poids de polyuréthane,
- de 0,01 à 2,0 % et de préférence de 0,01 à 1,0 % en poids de cellulose,
- de 0,05 à 5,0 % et de préférence de 0,1 à 3,0 % en poids de silicate de magnésium et d'aluminium.
En effet, une proportion de polyuréthane inférieure à 3 % ne permet pas d'obtenir un film suffisamment distinctif. L'utilisation dans la composition d'une proportion de cellulose supérieure à 1,0 % affecte le caractère pulvérisable de ladite composition et le film obtenu est fragile et cassant. Une proportion de silicate de magnésium et d'aluminium inférieure à 0, 1 % diminue l'efficacité de la mise en suspension des particules et une proportion supérieure à 3,0 % rend le film produit fragile.
La cellulose utilisée dans la composition selon l'invention peut être tout type de cellulose. De façon avantageuse, la cellulose contenue dans la composition selon l'invention est choisie parmi la méthylcellulose, l'hydroxypropyl méthylcellulose, l'éthylcellulose et l'hydroxyéthyl cellulose.

Comme indiqué précédemment, la composition filmogène selon l'invention est un nouveau système de délivrance d'un agent actif au niveau de la peau. Par conséquent, la composition filmogène selon l'invention contient un agent actif.
De préférence, cet agent actif est un agent thérapeutique ou un agent à usage cosmétique ou un mélange des deux.
De façon avantageuse, cet agent actif est un agent naturel ou synthétique, hydrophile ou lipophile.
Dans un mode plus particulier de mise en oeuvre de l'invention, ledit agent actif est choisi parmi les alpha- ou beta-hydroxy acides ou leurs dérivés; les extraits de plantes ou leurs dérivés; des vitamines telles que les vitamines C, B, E, K ou A ou les sels, les formes esters, alcoolates ou acides de celles-ci; les produits marins ou leurs dérivés; les hormones; les enzymes.
La composition filmogène selon l'invention contient environ de 0,01 à 10% en poids d'agent actif.

La composition filmogène selon l'invention peut contenir d'autres composés et notamment un ou plusieurs agents choisis parmi : les solvants hydrophiles, les solvants lipophiles, les humidifiants/plastifiants, les polymères épaississants autres que la cellulose et le polyuréthane, les colorants et pigments, les tensioactifs/émulsifiants, les parfums, les conservateurs, les agents chélatants, les absorbeurs d'UV, les antioxydants, les agents kératolytiques, le dihydroxy acétone et les agents favorisant la pénétration.

Les solvants hydrophiles utilisés dans la composition selon l'invention peuvent être tout solvant généralement utilisé en cosmétologie ou en dermatologie. On peut citer, à titre d'exemple et de façon non-limitative, comme solvant utilisable dans la composition selon l'invention, l'eau, des alcools comme l'éthanol, des glycols ou un mélange de ces composés. De façon avantageuse, la composition filmogène selon l'invention contient environ de 5 à 90 % en poids de solvants hydrophiles.

L'utilisation dans la composition selon l'invention de solvants lipophiles tels que des hydrocarbones ou des huiles est possible. On peut notamment citer à titre d'exemple et de façon non exhaustive, comme solvants lipophiles, l'iso-paraffine, des huiles minérales, l'isododécane, l'huile d'amande douce ou d'autres huiles naturelles. De préférence, la composition selon l'invention contient environ de 1 à 10 % en poids de solvants lipophiles.

Les humidifiants/plastifiants utilisés dans la composition selon l'invention pour modifier les propriétés du film et sa durée peuvent être tout ingrédient liant l'eau, généralement utilisé dans les produits cosmétiques ou dermatologiques tel que les glycols (glycérine, propylène glycol, 1,3-butylène glycol et polyéthylène glycols), sorbitol, sodium PCA, sodium citrate, diméthicone copolyol, cyclométhicone. La composition selon l'invention contient avantageusement environ de 0,5 à 20 % d'humidifiants/plastifiants.

Les polymères épaississants autres que la cellulose et le polyuréthane utilisés dans la composition selon l'invention pour modifier la viscosité, l'étalement, l'aspect du produit fini et la résistance du film peuvent être tout polymère ou tout épaississant généralement utilisé dans les produits cosmétiques ou dermatologiques comme, à titre d'exemple et de façon non-exhaustive, la gomme de xanthane, le PVA, le PVP, le carbomère ou des mélanges tels que le Simugel A (polyacrylate d'ammonium/isohexadecane/huile de castor PEG-40), Ceragel EZ-7 (polyacrylamide, polydecene, laureth-7), Sepigel 305 (polyacrylamide, C₁₃-14 isopraffine, laureth-7), Salcare SC-92 (polyquaternium 32, huile minérale). La composition selon l'invention contient environ de 0,1 à 10 % de polymères épaississants autres que la cellulose et le polyuréthane.

Les tensioactifs et les émulsifiants utilisés dans la composition selon l'invention pour stabiliser le produit fini ou pour améliorer son étalement sur la peau peuvent être tout émulsifiant ou tensioactif anionique, cationique ou non-ionique généralement utilisés dans les produits cosmétiques ou dermatologiques. Parmi ces tensioactifs et émulsifiants, on peut citer, à titre d'exemple et de façon non-limitative, les alcools éthoxylés, le sulfate de lauryl sodium, le polyquaternium-31. De façon avantageuse, la composition filmogène selon l'invention contient environ de 0,1 à 10 % de tensioactifs/émulsifiants.

Les colorants et les pigments dans la composition selon l'invention peuvent être tout colorant et pigment généralement utilisés dans les cosmétiques ou produits dermatologiques, voir même dans l'alimentation. On peut notamment citer, à titre d'exemples, le dioxyde de titane, les oxydes de zinc ou de fer. La composition filmogène selon l'invention contient environ de 0,01 à 12 % de colorants et pigments.

La composition selon l'invention peut avantageusement comprendre un parfum ou un mélange de parfums pour supporter un concept marketing ou pour masquer l'odeur naturelle de ladite composition. La composition selon l'invention contient, de préférence, environ de 0,1 à 5 % de parfums.

La composition selon l'invention peut également comprendre au moins un conservateur ou un mélange de conservateurs. Tout conservateur ou tout mélange de conservateurs connu de l'homme du métier dans le domaine des compositions cosmétiques ou dermatologiques est utilisable dans la composition de l'invention. L'utilisation d'un conservateur ou d'un mélange de conservateurs dans la composition selon l'invention permet de protéger ladite composition d'une contamination microbiologique. On peut notamment citer de façon non-exhaustive comme conservateur utilisable dans la composition le méthylparaben, le propylparaben, le phénoxyéthanol. La composition selon l'invention contient avantageusement environ de 0,01 à 3 % de conservateurs.

La composition selon l'invention peut également comprendre un agent chélatant ou un mélange d'agents chélatants pour lier les ions métalliques et pour aider à la stabilité globale de ladite composition. Tout agent chélatant utilisé dans les produits cosmétiques ou dermatologiques peut être ajouté à la composition comme, par exemple et de façon non-limitative, le EDTA, Na²EDTA, Na⁴EDTA. La composition filmogène selon l'invention contient environ de 0,01 à 1 % d'agents chélatants.

Des absorbeurs d'UV peuvent être utilisés dans la composition selon l'invention pour protéger la couleur de ladite composition des UV. Tout écran solaire, soluble dans l'eau ou dans l'huile, généralement connu de l'homme du métier est utilisable dans la composition selon l'invention tel que, par exemple et de façon non exhaustive, des filtres UV organiques comme le benzophénone-3, le benzophénone-4, l'octyl salicylate, l'octylméthoxy cinnamate ou des filtres inorganiques comme le dioxyde de titane ou l'oxyde de zinc. La composition filmogène selon l'invention contient environ de 0,01 à 10 % d'absorbeurs d'UV.

La composition selon l'invention peut comprendre des antioxydants afin de protéger ladite composition de l'oxydation. Tout oxydant ou mélange d'antioxydants, généralement utilisé dans les produits cosmétiques ou dermatologiques, est utilisable dans la composition selon l'invention. On peut notamment citer de façon non exhaustive comme antioxydant utilisable dans ladite composition le tocophérol, l'acétate de tocophérol, le gallate de propyle, le BHA ou le BHT. De façon avantageuse, la composition filmogène selon l'invention contient environ de 0,05 à 3 % en poids d'antioxydants.

La composition filmogène à usage topique, objet de l'invention se présente avantageusement sous une forme liquide ou sous une forme semi-liquide. De façon préférentielle, cette composition se présente sous la forme d'un spray, d'une lotion, d'un gel, d'un stick ou d'une mousse.

Lorsque la composition filmogène est appliquée sur la peau des utilisateurs, elle peut être conservée pendant une période de 20 min à plusieurs heures, sous la forme d'un film sec. De façon avantageuse, la composition appliquée sur la peau des utilisateurs peut être éliminée avec de l'eau ou avec un mélange d'eau et de tensioactifs.

La présente invention concerne également l'utilisation d'une composition filmogène à usage topique telle que décrite précédemment pour préparer un médicament destiné à traiter une affection dermatologique. Ladite affection dermatologique à traiter est choisie tout particulièrement parmi l'acné, le psoriasis et l'eczéma. L'agent actif utilisé dans la composition cosmétique utilisée pour son activité dans le traitement de ces affections dermatologiques.

La présente invention concerne également l'utilisation d'une composition filmogène à usage topique telle que décrite précédemment pour préparer un produit cosmétique.

D'autres avantages et caractéristiques de l'invention apparaîtront à la lecture des exemples qui suivent concernant des formulations de compositions filmogènes à usage topique et leur procédé de préparation. Ces exemples sont donnés à titre illustratif et ne sauraient limiter la portée de l'invention.

### I. Exemples de formulation de compositions filmogènes à usage topique, objet de la présente invention.

Dans le tableau 1 ci-dessous, sont présentées différentes formulations de compositions filmogènes à usage topique, objet de la présente invention. La formulation A correspond à une composition filmogène telle que définie précédemment contenant des absorbeurs d'UV.
Les formulations B et C correspondent à une composition filmogène contenant comme agent actif un extrait de plantes. La formulation C, contrairement à la formulation B, ne contient pas d'alcool car l'extrait de plantes utilisé dans cette formulation perd toute activité en présence d'alcool.

| **Ingrédients** | **A** (% wt/wt) | **B** (% wt/wt) | **C** (% wt/wt) |
|---|---|---|---|
| Eau | qsp 100 | qsp 100 | qsp 100 |
| Méthylcellulose | 0,01 à 1,0 | 0,01 à 1,0 | 0,01 à 1,0 |
| Silicate de Mg, Al | 0,10 à 3,0 | 0,10 à 3,0 | 0,10 à 3,0 |
| Polyuréthane | 3,0 à 30,0 | 3,0 à 30,0 | 3,0 à 30,0 |
| Alcool | 0,1 à 30,0 | 0,1 à 30,0 | - |
| Glycol (butylène, propylène, hexalène ou glycérine ou PEG) | 0,10 à 5,0 | 0,1 à 5,0 | 0,1 à 10,0 |
| Filtres inorganiques (dioxyde de titane ou oxydes de fer) | 0,01 à 10,0 | - | - |
| Absorbeurs d' UV (benzophenone 3 ou 4, octyl salicylate, octyl méthoxycinnamate) | 1,0 à 8,0 | - | - |
| Conservateurs | 0,01 à 3,0 | 0,01 à 3,0 | 0,01 à 3,0 |
| Agent actif | | 0,01 à 10,0 | 0,01 à 10,0 |
| Agent modifiant la viscosité | 0,01 à 3,0 | 0,01 à 3,0 | 0,01 à 3,0 |
| Agent ajustant le pH (amines ou hydroxydes, acides organiques ou inorganiques) | 0,01 à 3,0 | 0,01 à 3,0 | 0,01 à 3,0 |

### II. Préparation de compositions filmogènes, objet de la présente invention.

Les polymères sont mélangés dans l'eau ou dans l'alcool ou dans un mélange de ceux-ci. Un apport de chaleur peut être nécessaire si de l'argile ou une gomme sont utilisées. Les polymères sont ajoutés l'un après l'autre et mélangés jusqu'au lissage du mélange en utilisant un mixeur standard à une vitesse choisie en fonction du mélange. Après avoir obtenu un mélange uniforme de polymères, les autres ingrédients sont ensuite ajoutés. Une grande vitesse de mélange doit être utilisée pour complètement incorporer les filtres UV dans la préparation.

### III. Autres exemples de formulation de compositions filmogènes à usage topique, objet de la présente invention.

Le tableau 2 ci-dessous présente deux autres exemples (exemples 4 et 5) de formulation de compositions filmogènes à usage topique objet de la présente invention.

**Tableau 2**

| **Ingredients** | **Exemple 4** (% w/w) | **Exemple 5** (% w/w) |
|---|---|---|
| Eau | Qsp 100 | Qsp 100 |
| Glycérine | 1,0 | |
| Biophile H (Lectine hydrogénée, Alcools C_{12-16'} acide palmitique) | 2,0 | |
| Hydroxypropyl méthylcellulose | 0,20 | 0,20 |
| Silicate de magnésium et d'aluminium | 0,45 | 0,30 |
| Carbomère | 0,15 | 0,15 |
| Amino méthylpropanol | 0,20 | 0,20 |
| Polyuréthane | 20,0 | 10,0 |
| Diméthicone Copolyol | 0,50 | 0,50 |
| Conservateur | 0,70 | |
| Ascorbyl phosphate de magnésium | - | 0,20 |
| Ethanol SDA 40-2 | - | 9,0 |

La composition identifiée dans le tableau 2 comme l'exemple 4 peut être préparée comme suit :
(a) le biophile H (obtenu auprès de Lucas Meyer) est mélangé avec de l'eau sous agitation lente à 70°C;
(b) la glycérine est ajoutée au produit obtenu à l'étape (a) sous agitation lente continue jusqu'à l'obtention d'une dispersion grossière et fluide; il faut prendre soin durant cette étape de garder la température au-dessus de 50°C;
(c) l'hydroxypropyl méthylcellulose (méthocel E4M obtenu auprès de Dow Chemical) est ajoutée au produit obtenu à l'étape (b) en les mélangeant de façon continue et ce, jusqu'à ce qu'elle soit complétement hydratée;
(d) le silicate de magnésium et d'aluminium (Veegum HV obtenu auprès de R.T.Vandrbilt) est ajouté au produit obtenu à l'étape (c) en les mélangeant de façon continue et ce, jusqu'à ce qu'il soit complétement hydraté;
(e) le carbomère (obtenu auprès de Noveon) est ajouté au produit obtenu à l'étape (d) en les mélangeant de façon continue et ce, jusqu'à ce qu'il soit complétement hydraté;
(f) l'amino méthylpropanol (obtenu auprès de Angus) est ajouté au produit obtenu à l'étape (e) en les mélangeant de façon continue et ce, jusqu'à ce qu'un produit uniforme lisse soit obtenu;
(g) le polyuréthane est ajouté au produit obtenu à l'étape (f) en les mélangeant parfaitement;
(h) le diméthicone copolyol est ajouté au produit obtenu à l'étape (g) en les mélangeant parfaitement; et,
(i) le conservateur est ajouté au produit obtenu à l'étape (h) en les mélangeant parfaitement.

La composition identifiée dans le tableau 2 comme l'exemple 5 peut être préparée comme suit :
(a) l'hydroxypropyl méthylcellulose (méthocel E4M obtenu auprès de Dow Chemical) est mélangée avec de l'eau jusqu'à complète hydratation;
(b) le silicate de magnésium et d'aluminium (Veegum HV obtenu auprès de R.T.Vandrbilt) est ajouté au produit obtenu à l'étape (a) jusqu'à complète hydratation;
(c) le carbomère est ajouté au produit obtenu à l'étape (b) jusqu'à complète hydratation;
(d) le produit obtenu à l'étape (c) est chauffé jusqu'à environ 50°C;
(e) l'amino méthylpropanol est ajouté au produit obtenu à l'étape (d) en les mélangeant de façon continue et ce, jusqu'à ce qu'un produit uniforme lisse soit obtenu;
(f) l'ascorbyl phosphate de magnésium est dissous danbs de l'eau et chauffé jusqu'à environ 40°C;
(g) le produit obtenu à l'étape (f) est mélangé avec le produit obtenu à l'étape (e);
(h) le polyuréthane est ajouté au produit obtenu à l'étape (g) sous agitation continue;
(i) le diméthicone copolyol est ajouté au produit obtenu à l'étape (h) sous agitation continue;
(j) l'éthanol CDA 40-2 est ajouté au produit obtenu à l'étape (i) sous agitation continue; et,
(k) le conservateur est ajouté est ajouté au produit obtenu à l'étape (j) sous agitation continue jusqu'à ce qu'un produit uniforme soit obtenu.

### Référence:

International Cosmetic Ingredient Dictionary and Handbook, Eighth Edition
Harry's Cosmetology
Remington's Pharmaceutical Sciences

## Revendications

1. Composition filmogène à usage topique **caractérisée en ce qu'**elle contient au moins:
- de 1 à 50 % de polyuréthane-1, ledit polyuréthane étant un copolymère de monomères d'acide isophtalique, d'acide adipique, de glycol héxylène, de glycol néopenthyl, d'acide diméthylolpropanique et de diisocyanate d'isophorone ;
- de 0,01 à 2,0 % en poids de cellulose ;
- de 0,05 à 5,0 % en poids de silicate de magnésium et d'aluminium.

2. Composition selon la revendication 1, **caractérisée en ce que** ladite cellulose est choisie parmi la méthylcellulose, l'hydroxypropyl méthylcellulose, l'éthylcellulose et l'hydroxyéthyl cellulose.

3. Composition selon l'une quelconque des revendications 1 à 2, **caractérisée en ce qu'**elle contient au moins un agent actif.

4. Composition selon la revendication 3, **caractérisée en ce que** ledit agent actif est un agent thérapeutique ou un agent à usage cosmétique ou un mélange des deux.

5. Composition selon l'une quelconque des revendications 3 ou 4, **caractérisée en ce que** ledit agent actif est un agent naturel ou synthétique hydrophile ou lipophile.

6. Composition selon l'une quelconque des revendications 3 à 5, **caractérisée en ce que** ledit agent actif est choisi parmi les alpha- ou beta-hydroxy acides ou leurs dérivés; les extraits de plantes ou leurs dérivés; des vitamines telles que les vitamines C, B, E, K ou A ou les sels, les formes esters, alcoolates ou acides de celles-ci; les produits marins ou leurs dérivés; les hormones; les enzymes.

7. Composition selon l'une quelconque des revendications 3 à 6, **caractérisée en ce qu'**elle contient environ de 0,01 à 10% en poids d'agent actif.

8. Composition selon l'une quelconque des revendications précédentes, **caractérisée en ce qu'**elle contient en outre un ou plusieurs agents choisis parmi : les solvants hydrophiles, les solvants lipophiles, les humidifiants/plastifiants, les polymères épaississants autres que la cellulose et le polyuréthane, les colorants et pigments, les tensioactifs/émulsifiants, les parfums, les conservateurs, les agents chélatants, les absorbeurs d'UV, les antioxydants, les agents kératolytiques, le dihydroxy acétone et les agents favorisant la pénétration.

9. Composition selon la revendication 8, **caractérisée en ce qu'**elle contient environ de 5 à 90 % en poids de solvants hydrophiles.

10. Composition selon l'une quelconque des revendications 8 ou 9, **caractérisée en ce qu'**elle contient environ de 1 à 10 % en poids de solvants lipophiles.

11. Composition selon l'une quelconque des revendications 8 à 10, **caractérisée en ce qu'**elle contient environ de 0,5 à 20 % de humidifiants/plastifiants.

12. Composition selon l'une quelconque des revendications 8 à 11, **caractérisée en ce qu'**elle contient environ de 0,1 à 10 % de polymères épaississants autres que la cellulose et le polyuréthane.

13. Composition selon l'une quelconque des revendications 8 à 12, **caractérisée en ce qu'**elle contient environ de 0,01 à 12 % de colorants et pigments.

14. Composition selon l'une quelconque des revendications 8 à 13, **caractérisée en ce qu'**elle contient environ de 0,1 à 10 % de tensioactifs/émulsifiants.

15. Composition selon l'une quelconque des revendications 8 à 14, **caractérisée en ce qu'**elle contient environ de 0,1 à 5 % de parfums.

16. Composition selon l'une quelconque des revendications 8 à 15, **caractérisée en ce qu'**elle contient environ de 0,01 à 3 % de conservateurs.

17. Composition selon l'une quelconque des revendications 8 à 16, **caractérisée en ce qu'**elle contient environ de 0,01 à 1 % d'agents chélatants.

18. Composition selon l'une quelconque des revendications 8 à 17, **caractérisée en ce qu'**elle contient environ de 0,01 à 10 % d'absorbeurs d'UV.

19. Composition selon l'une quelconque des revendications 8 à 18, **caractérisée en ce qu'**elle contient environ de 0,05 à 3 % en poids d'antioxydants.

20. Composition selon l'une quelconque des revendications précédentes, **caractérisée en ce qu'**elle se présente sous une forme liquide ou sous une forme semi-liquide.

21. Composition selon l'une quelconque des revendications précédentes, **caractérisée en ce qu'**elle se présente sous la forme d'un spray, d'une lotion, d'un gel, d'un stick ou d'une mousse.

22. Composition selon l'une quelconque des revendications précédentes, **caractérisée en ce qu'**elle peut être conservée sur la peau des utilisateurs pendant une période de 20 min à plusieurs heures.

23. Composition selon l'une quelconque des revendications précédentes, **caractérisée en ce qu'**elle s'élimine de sur la peau des utilisateurs avec de l'eau ou avec un mélange d'eau et de tensioactifs.

24. Utilisation d'une composition filmogène à usage topique selon l'une quelconque des revendications 3 à 23, pour préparer un médicament destiné à traiter une affection dermatologique.

25. Utilisation selon la revendication 24, **caractérisée en ce que** ladite affection dermatologique à traiter est choisie parmi l'acné, le psoriasis et l'eczéma.

26. Utilisation d'une composition filmogène à usage topique selon l'une quelconque des revendications 1 à 23, pour préparer un produit cosmétique.

## Claims

1. Film-forming composition for topical use, **characterised in that** it contains at least:
- from 1 to 50% polyurethane-1, said polyurethane being a copolymer of monomers of isophthalic acid, adipic acid, hexylene glycol, neopentyl glycol, dimethylolpropanic acid and isophoron diisocyanate;
- from 0.01 to 2.0 % by weight of cellulose;
- from 0.05 to 5.0% by weight of magnesium and aluminium silicate.

2. Composition according to claim 1, **characterised in that** said cellulose is chosen among the methylcellulose, the hydroxypropyl methylcellulose, the ethylcellulose and the hydroxyethyl cellulose.

3. Composition according to anyone of claims 1 to 2, **characterised in that** it contains at lease one active agent.

4. Composition according to claim 3, **characterised in that** said active agent is a therapeutic agent or an agent for cosmetic use or a mixture of both.

5. Composition according to anyone of claims 3 or 4, **characterised in that** said active agent is a natural or synthetic hydrophilic or lipophilic agent.

6. Composition according to anyone of claims 3 to 5, **characterised in that** said active agent is chosen among alpha- or beta-hydroxy acids or their derivatives; plant extracts or their derivatives; vitamins such as vitamins C, B, E, K or A or the salts, the ester, alcoholate or acid forms thereof; the marine products or their derivatives; the hormones; the enzymes.

7. Composition according to anyone of claims 3 to 6, **characterised in that** it contains approximately from 0.01 to 10% by weight of active agent.

8. Composition according to anyone of the previous claims, **characterised in that** it further contains one or more agents chosen among: the hydrophilic solvents, the lipophilic solvents, the moisturisers/plasticizers, the thickening polymers other than the cellulose and the polyurethane, the colorants, and the pigments, the surfactants/emulsifiers, the fragrances, the preservatives, the chelating agents, the UV absorbers, the antioxidants, the keratolytic agents, dihydroxyacetone and penetration-promoting agents.

9. Composition according to claim 8, **characterised in that** it contains approximately from 5 to 90% by weight of hydrophilic solvents.

10. Composition according to anyone of claims 8 or 9, **characterised in that** it contains approximately from 1 to 10% by weight of lipophilic solvents.

11. Composition according to anyone of claims 8 to 10, **characterised in that** it contains approximately from 0.5 to 20% moisturisers/plasticizers.

12. Composition according to anyone of claims 8 to 11, **characterised in that** it contains approximately from 0.1 to 10% thickening polymers other than the cellulose and the polyurethane.

13. Composition according to anyone of claims 8 to 12, **characterised in that** it contains approximately from 0.01 to 12% colorants and pigments.

14. Composition according to anyone of claims 8 to 13, **characterised in that** it contains approximately from 0.1 to 10% surfactants/emulsifiers.

15. Composition according to anyone of claims 8 to 14, **characterised in that** it contains approximately from 0.1 to 5% fragrances.

16. Composition according to anyone of claims 8 to 15, **characterised in that** it contains approximately from 0.01 to 3% preservatives.

17. Composition according to anyone of claims 8 to 16, **characterised in that** it contains approximately from 0.01 to 1% chelating agents.

18. Composition according to anyone of claims 8 to 17, **characterised in that** it contains approximately from 0.01 to 10% UV absorbers.

19. Composition according to anyone of claims 8 to 18, **characterised in that** it contains approximately from 0.05 to 3% by weight of antioxidants.

20. Composition according to anyone of the previous claims, **characterised in that** it is in a liquid or semiliquid form.

21. Composition according to anyone of the previous claims, **characterised in that** it is in the form of a spray, a lotion, a gel, a stick or a foam.

22. Composition according Lo anyone of the previous claims, **characterised in that** it can be retained on the users' skin for a period of 20 min. to several hours.

23. Composition according to anyone of the previous claims, **characterised in that** it is removed from the users' skin with water or with a mixture of water and surfactants.

24. Use of a film-forming composition for topical use according to anyone of claims 3 to 23, in order to prepare a medication intended to treat a dermatological disease.

25. Use according to claim 24, **characterised in that** said dermatological disease to be treated is chosen among acne, psoriasis and eczema.

26. Use of a film-forming composition for topical use according to anyone of claims 1 to 23, in order to prepare a cosmetic product.

## Patentansprüche

1. Filmbildende Zusammensetzung zur topischen Anwendung, **dadurch gekennzeichnet, dass** sie mindestens enthält:
- von 1 bis 50 % Polyurethan-1, wobei das besagte Polyurethan ein Copolymer von Monomeren von Isophtalsäure, von Adipinsäure, von Hexylenglykol, von Neopenthylglykol, von Dimethylolpropansäure und von Isophorondiisocyanat ist;
- 0,01 bis 2,0 Gew.-% Zellulose,
- 0,05 bis 5,0 Gew.-% Magnesium- und Aluminiumsilikat.

2. Zusammensetzung nach Anspruch 1, **dadurch gekennzeichnet, dass** die besagte Zellulose ausgewählt ist aus der Methylzellulose, der Hydroxypropylmethylzellulose, der Ethyzellulose und der Hydroxyethylzellulose.

3. Zusammensetzung nach einem der Ansprüche 1 bis 2, **dadurch gekennzeichnet, dass** sie mindestens einen aktiven Wirkstoff enthält.

4. Zusammensetzung nach Anspruch 3, **dadurch gekennzeichnet, dass** der besagte aktive Wirkstoff ein therapeutischer Wirkstoff oder ein Wirkstoff zur kosmetischen Anwendung oder ein Gemisch der beiden ist.

5. Zusammensetzung nach einem der Ansprüche 3 oder 4, **dadurch gekennzeichnet, dass** der besagte aktive Wirkstoff ein natürlicher oder synthetischer hydrophiler oder lipophiler Wirkstoff ist.

6. Zusammensetzung nach einem der Ansprüche 3 bis 5, **dadurch gekennzeichnet, dass** der besagte aktive Wirkstoff ausgewählt ist aus den Alpha- oder Beta-Hydroxysäuren oder deren Derivaten; den Pflanzenextrakten oder deren Derivaten; den Vitaminen wie zum Beispiel den Vitaminen C, B, E, K oder A oder den Salzen, den Esterformen, Alkoholaten oder Säuren derselben; den Meeresprodukten oder deren Derivaten, den Hormonen; den Enzymen.

7. Zusammensetzung nach einem der Ansprüche 3 bis 6, **dadurch gekennzeichnet, dass** sie zirka 0,01 bis 10 Gew.-% aktiven Wirkstoff enthält.

8. Zusammensetzung nach einem der vorangehenden Ansprüche, **dadurch gekennzeichnet, dass** sie außerdem einen oder mehrere Wirkstoffe enthält, ausgewählt aus: den hydrophilen Lösungsmitteln, den lipophilen Lösungsmitteln, den Feuchthaltemitteln/Weichmachern, den anderen Verdickungsmitteln als Zellulose und Polyurethan, den Farbstoffen und Pigmenten, den Tensiden/Emulgatoren, den Duftstoffen, den Konservierungsstoffen, den Chelatbildnern, den UV-Absorbern, den Antioxidantien, den keratolytischen Wirkstoffen, dem Dihydroxyaceton und den Wirkstoffen, die das Eindringen fördern.

9. Zusammensetzung nach Anspruch 8, **dadurch gekennzeichnet, dass** sie zirka 5 bis 90 Gew.-% hydrophile Lösungsmittel enthält.

10. Zusammensetzung nach einem der Ansprüche 8 oder 9, **dadurch gekennzeichnet, dass** sie zirka 1 bis 10 Gew.-% lipophile Lösungsmittel enthält.

11. Zusammensetzung nach einem der Ansprüche 8 bis 10, **dadurch gekennzeichnet, dass** sie zirka 0,5 bis 20 % Feuchthaltemittel/Weichmacher enthält.

12. Zusammensetzung nach einem der Ansprüche 8 bis 11, **dadurch gekennzeichnet, dass** sie zirka 0,1 bis 10 % verdickende Polymere enthält, die keine Zellulose und kein Polyurethan sind.

13. Zusammensetzung nach einem der Ansprüche 8 bis 12, **dadurch gekennzeichnet, dass** sie zirka 0,01 bis 12 % Farbstoffe und Pigmente enthält.

14. Zusammensetzung nach einem der Ansprüche 8 bis 13, **dadurch gekennzeichnet, dass** sie zirka 0,1 bis 10 % Tenside/Emulgatoren enthält.

15. Zusammensetzung nach einem der Ansprüche 8 bis 14, **dadurch gekennzeichnet, dass** sie zirka 0,1 bis 5 % Duftstoffe enthält.

16. Zusammensetzung nach einem der Ansprüche 8 bis 15, **dadurch gekennzeichnet, dass** sie zirka 0,01 bis 3 % Konservierungsstoffe enthält.

17. Zusammensetzung nach einem der Ansprüche 8 bis 16, **dadurch gekennzeichnet, dass** sie zirka 0,01 bis 1 % Chelatbildner enthält.

18. Zusammensetzung nach einem der Ansprüche 8 bis 17, **dadurch gekennzeichnet, dass** sie zirka 0,01 bis 10 % UV-Absorber enthält.

19. Zusammensetzung nach einem der Ansprüche 8 bis 18, **dadurch gekennzeichnet, dass** sie zirka 0,05 bis 3 Gew.-% Antioxidantien enthält.

20. Zusammensetzung nach einem der vorangehenden Ansprüche, **dadurch gekennzeichnet, dass** sie eine flüssige oder eine halbflüssige Form aufweist.

21. Zusammensetzung nach einem der vorangehenden Ansprüche, **dadurch gekennzeichnet, dass** sie die Form eines Sprays, einer Lotion, eines Gels, eines Sticks oder eines Schaums aufweist.

22. Zusammensetzung nach einem der vorangehenden Ansprüche, **dadurch gekennzeichnet, dass** sie auf der Haut der Benutzer während eines Zeitraums von 20 min. bis zu mehreren Stunden konservierbar ist.

23. Zusammensetzung nach einem der vorangehenden Ansprüche, **dadurch gekennzeichnet, dass** sie von der Haut der Benutzer mit Wasser oder einem Gemisch aus Wasser und Tensiden zu entfernen ist.

24. Verwendung einer filmbildenden Zusammensetzung zur topischen Anwendung nach einem der Ansprüche 3 bis 23 zur Zubereitung eines Medikaments, das zur Behandlung einer dermatologischen Erkrankung bestimmt ist.

25. Verwendung nach Anspruch 24, **dadurch gekennzeichnet, dass** die besagte dermatologische Erkrankung, die zu behandeln ist, ausgewählt ist aus der Akne, der Psoriasis und dem Ekzem.

26. Verwendung einer filmbildenden Zusammensetzung zur topischen Anwendung nach einem der Ansprüche 1 bis 23 zur Zubereitung eines kosmetischen Produckt.
